Europäisches Patentamt

European Patent Office

Office européen des brevets

(11) Publication number: **0 386 092 B1**

# EUROPEAN PATENT SPECIFICATION

(45) Date of publication of patent specification: **29.06.94**

(51) Int. Cl.⁵: **G01N 33/68**, G01N 33/544, //G01N33/574,G01N33/80, G01N33/564

(21) Application number: **88910068.1**

(22) Date of filing: **10.11.88**

(86) International application number: **PCT/GB88/00978**

(87) International publication number: **WO 89/04490 (18.05.89 89/11)**

(54) **HUMAN IgG GLYCOSYLATION ASSAY.**

(30) Priority: **10.11.87 GB 8726271**

(43) Date of publication of application:
**12.09.90 Bulletin 90/37**

(45) Publication of the grant of the patent:
**29.06.94 Bulletin 94/26**

(84) Designated Contracting States:
**AT BE CH DE FR GB IT LI LU NL SE**

(56) References cited:
**EP-A- 0 057 236
EP-A- 0 255 342**

**CHEMICAL ABSTRACTS, vol. 108, no. 7, 15 February 1988, Columbus, OH (US); R. LOTAN et al., p. 610, abstract no. 54738p&NUM;**

**CHEMICAL ABSTRACTS, vol. 104, no. 7, 17 February 1986; Columbus, OH (US); D.L. RUS-SEL et al., p. 294, abstract no. 48407f&NUM;**

(73) Proprietor: **G.D. Searle & Co.
P.O. Box 5110
Chicago Illinois 60680(US)**

(72) Inventor: **SUMAR, Nazira, Middlesex School of Medicine
Dept. of Immun. & Rheumat. Res.,
A. Stanley House
40-50 Tottenham Street, London W1P 9PG(GB)**
Inventor: **BODMAN, Katherine, Middlesex School of Med.
Dept. of Immun. & Rheumat. Res.,
A. Stanley House
40-50 Tottenham Street, London W1P 9PG(GB)**
Inventor: **HAY, Frank, St.George's Hospital Med. School
Division of Immunology,
Cranmer Terrace
London SW17 0RE(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

BIOLOGICAL ABSTRACTS, vol. 79, no. 6, 1985, Philadelphia, PA (US); D.L. DEANE et al., p. AB-594, abstract no. 51072&NUM;

NATURE, vol. 316, 01 August 1985; R.B. PAREKH et al., pp. 452-457&NUM;

BIOLOGICAL ABSTRACTS, vol. 84, no. 5, 1987, Philadelphia, PA (US); M.G. MALAISE et al., p. AB-505, abstract no. 46728&NUM;

JOURNAL EXPERIMENTAL MEDICINE, vol. 167, 1988; pp. 1731-1736&NUM;

Inventor: **ROITT, Ivan, Middlesex School of Med.**
**Dept. of Immun. & Rheumat. Res.,**
**A. Stanley House**
**40-50 Tottenham Street, London W1P 9PG(GB)**
Inventor: **PAREKH, Raj, Oxford Oligosaccharide Group**
**Dept. of Biochemistry,**
**University of Oxford**
**South Parks Road, Oxford OX1 3OU(GB)**
Inventor: **RADEMACHER, Thomas, Oxford Oligosaccharide Group**
**Dept. of Biochemistry,**
**University of Oxford**
**South Parks Road, Oxford OX1 3OU(GB)**
Inventor: **DWEK, Raymond, Oxford Oligosaccharide Group**
**Dept. of Biochemistry,**
**University of Oxford**
**South Parks Road, Oxford OX1 3OU(GB)**

(74) Representative: **Collier, Jeremy Austin Grey et al**
**J.A.KEMP & CO.**
**14, South Square**
**Gray's Inn**
**London WC1R 5LX (GB)**

EP 0 386 092 B1

**Description**

This invention relates to the assay of glycosylated proteins and more particularly to detecting and/or measuring the terminal glycosylation patterns of proteins.

It is known that in some pathological conditions the terminal sugar residues of glycosylated proteins are altered. For example, it has been shown (Parekh et al, Nature 316 pp 452-457, 1985, and European Patent Publication no. 189388) that, in rheumatoid arthritis, serum IgG shows a reduced content of terminal galactose residues and a correspondingly increased content of terminal N-acetylglucosamine residues. The content of the latter in the protein is not increased, but the absence of galactose residues causes the N-acetylglucosamine residues to become the terminal sugar residues. Malaise et al, Clin. exp. Immunol. (1987) 68 pp 543-551 have described a solid phase radioimmunoassay for direct measurement of the binding capacity of human IgG for the lectins peanut agglutinin, Concanavalin A and pokeweed mitogen which bind mainly to $\beta$-galactose, alpha-mannose and N-acetyl-$\beta$-glucosamine respectively. It was found that the mean specific binding of IgG isolated from the serum of patients with clinically diagnosed rheumatoid arthritis to Concanavalin A was significantly higher than that of normal IgG.

It has also been shown (Leathem et al, The Lancet of May 9th, 1987 pp 1054-1057) that the prognosis for sufferers from breast cancer may be related to the ability of the cancer cells to bind a lectin specific for N-acetylgalactosamine residues. Cancer cells which bind this lectin were found to be associated with a propensity to metastasise to local lymph nodes. Conversely, cancer cells which did not bind to this lectin had a much lower tendency to form metastases. Other important carbohydrate alterations, detectable with lectins, occur in spina bifida, cystic fibrosis and prostatic cancer. Such alterations can also be significant in the detection of (sometimes rare) blood groups, and changes in acute phase proteins in inflammation.

It is therefore of interest to be able to determine in a simple, cheap and rapid way the glycosylation patterns of proteins such as IgG and cell surface proteins. In practice, the terminal sugar residues likely to be of interest in many cases are those already noted, namely galactose (or N-acetyl-galactosamine) and N-acetylglucosamine (GlcNac). The former provides the usual terminal sugar residues in many cases, but the latter become the terminal residues when the glycosylation pattern of the protein in question is abnormal. In cystic fibrosis, terminal fucose residues are reduced with a corresponding increase in terminal N-acetyl-glucosamine residues.

The methods heretofore described for the detection and measurement of terminal glycosylation patterns have not been entirely satisfactory more particularly because they are complicated and expensive to operate. Thus Parekh et al (Nature 316 pp 452-457, 1985) used a method based on chromatography of oligosaccharides isolated from the IgG of rheumatoid arthritis patients on an agarose column to which the lectin from Ricinus communis was bound, or an enzymatic method (J.Exp. Med., 167, pp 1731-1736, 1988). The method of Leatham et al involved incubating sections of cancer tissue with the lectin from the snail Helix pomatia (which binds to N-acetyl galactosamine residues), and adding rabbit anti-serum to this lectin followed by biotinylated swine anti-rabbit immunoglobulin, and avidin-peroxidase. Peroxidase activity was then revealed using hydrogen peroxide and diamino benzidine. These methods are not simple.

We have now devised a simple means for detecting and/or measuring the glycosylation pattern of the terminal sugar residues of the oligosaccharide of a protein. The new method is rapid and simple to operate and uses readily available reagents. It is essentially based on the idea that what is required is not an absolute measure of the content of a particular possible terminal sugar residue, but rather an estimate of the relative proportions of the different terminal sugars which may potentially be present.

The method of the invention for detecting and/or measuring the terminal glycosylation pattern of human IgG is characterised in that it comprises treating samples of IgG, which have been treated to expose terminal sugar residues, with a corresponding plural number of labelled lectins at least one of which has a specific binding affinity for galactose and at least one other of which has a specific binding affinity for N-acetylglucosamine, each such lectin being used in excess, removing unbound labelled lectin from the said samples, and then observing and/or measuring the signal provided by each bound or unbound lectin. By carrying out the method of samples of IgG of known comparability, e.g. identical samples or samples which differ only in their dilution ratio, what is observed is the difference in the ability of the samples of IgG to bind the labelled lectins which are specific for the galactose and N-acetylglucosamine residues to be determined.

The reagents having specific binding affinity for the terminal galactose and N-acetylglucosamine residues are lectins. These are proteins of plant or animal origin which have specific binding affinity for particular sugar residues. Many lectins are known and they may be obtained commercially. They may be labelled by any of the methods customarily used for labelling proteins which are used in assay techniques based on specific binding affinities, e.g. by incorporation of radioactive atoms, or by binding, directly or

3

indirectly, to enzymes. Enzymatic labelling is usually preferred as it avoids the need for the special equipment associated with the use of radioactive materials.

The new method is especially useful for measuring the proportion of IgG molecules lacking the normal terminal galactose residues and having, as a consequence, terminal N-acetylglucosamine residues. As noted above, the relative absence of terminal galactose residues is characteristic of the IgG of patients suffering from rheumatoid arthritis and the presence of terminal N-acetylgalactosamine residues in the cell surface proteins of breast cancer tissue has been found to be associated with a tendency of the cancer to metastasise.

The new method is conveniently operated by making use of the ability of IgG to bind strongly to certain solid substrates, especially nitrocellulose. Other solid substrates can be based on nylon (especially nylon-66, available from Amersham) and specially treated paper, especially amino-thiophenol treated paper available as Transbind APT (Trade Mark) paper. Suitable samples of the IgG to be examined, after an appropriate purification treatment, are bound to the solid substrate. The IgG should be denatured either by boiling after binding to the solid phase or by disulphide bond reduction and treatment with 8M urea before binding to the solid phase. Denaturation appears to be essential.

The bound samples are then treated with labelled lectins chosen for their specificity to the terminal galactose and N-acetylglucosamine residues whose presence or absence is to be detected. The signals provided by the labels are then observed or measured, usually after appropriate development, e.g. with a colour forming reagent which is activated by the enzyme used as label, and the ratio of the signals provided by the labelled lectins which react with, respectively, the normal terminal sugar residues (galactose) and the abnormal terminal sugar residues (N-acetylglucosamine), gives directly a measure of the latter. It should be noted in this connection that an absolute determination of the content of a particular terminal sugar residue is not required. All that is necessary is that the reagents used should be calibrated against known standards and that the samples of IgG bound to the solid support should all be the same or have a known relationship to one another in terms of the quantity of IgG.

The labelled lectins used in the new method may readily be prepared from commercially available lectins and commercially available labelling reagents. Suitable reagents are mentioned in the detailed description of the new method given below, but it will be understood that these reagents may be replaced by other reagents fulfilling the same function.

In the currently preferred method for operating the invention, IgG is isolated from blood samples in known manner and made up into a solution of known IgG content. This solution is then dot blotted onto a nitrocellulose sheet in carefully controlled quantity. The IgG binds tightly to the nitrocellulose. For dot blotting the samples, commercial dot blotters can be used. The assay may also be used on mixtures of proteins run on SDS-PAGE gels and western blotted onto nitrocellulose paper. The blots can then be treated in the manner described herein. In that way, the IgG in a range of proteins can be examined simultaneously. The method can however be applied to the estimation of the terminal glycosylation pattern, using lectins, using any type of reaction in a liquid medium or any type of solid phase support such as plastic microtitre plates, for example polystyrene ELISA plates such as those sold by Nunc or Dynatech.

In the preferred method, the dot blotted IgG is treated with a buffer containing a wetting agent and an inert protein, e.g. bovine serum albumin, to prevent further non-specific binding of protein to the nitrocellulose. The dots are then treated with the labelled lectin. The lectin may be, for example, biotinylated by reaction with N-hydroxysuccinimidobiotin. After washing to remove unbound lectin, the dots are treated with avidin having peroxidase (or other suitable enzyme) bound thereto. After further washing, the peroxidase, or other enzyme label, is detected by reaction with a suitable colour forming reagent, e.g. a combination of chloro-naphthol and hydrogen peroxide. The colour produced may be measured with an optical densitometer of the kind used in known enzyme linked immunoassay analysis. Alternatively the avidin may be labelled with a radio-isotope e.g. as $^{125}$I-streptavidin. The bound radioactivity may then be measured directly.

Lectins from Abrus precatorius (abrin) and Ricinus communis (ricin; RCAI or RCAII B chain), are suitable for binding to terminal galactose residues, while the lectins from Bandeiraea simplicifolia II (Griffonia simplicifolia II), Datura stramonium, or Lyconersicon esculentum or succinylated wheat germ agglutinin, are suitable for binding to terminal N-acetylglucosamine residues. The Bandeiraea simplicifolia II (Griffonia simplificolia II) lectin is of particular interest because it is the only known lectin that interacts solely with terminal non-reducing $\alpha$- and $\beta$- N-acetylglucosamine residues.

The following Examples illustrate the invention.

EXAMPLE 1

IgG purification

Blood samples were incubated at 37°C for one hour to allow clotting. They were then centrifuged at 3000 rpm for 25 minutes and the serum was removed and stored at -20°C. IgG was isolated from the serum (0.5 to 2 ml) in the following way. All the operations were carried out at room temperature. Half a volume of saturated ammonium sulphate solution at pH 7.2 was added dropwise with stirring to one volume of serum and the mixture was allowed to stand for 25 minutes and then centrifuged at 3000 rpm for 25 minutes. The precipitated protein pellet was resuspended in the original serum volume of 40% ammonium sulphate solution, and then recentrifuged at 3000 rpm for 25 minutes. The protein pellet obtained was again suspended in the original serum volume of 20 mM potassium phosphate buffer at pH 7.2 (KPi). The solution obtained was dialysed against 20 mM KPi and then purified on a column of DEAE anion exchange cellulose (DE52, N about 8 g of gel in a 5 ml syringe barrel equilibrated with 20 mM KPi at pH 7.2; 2-5 g DEAE cellulose/ml of serum). The dialysed IgG solution (0.5 - 2 ml) was added to the column and eluted with KPi. The following fractions were collected:

| (1) | 2 ml |
|-----|------|
| (2) | 1 ml |
| (3) | 4 ml |
| (4) | 1 ml |
| (5) | 5 ml |

The IgG elutes in fractions (2) and (3). (This was checked by spectrophotometric examination at 280 nm.) The IgG-containing fractions were pooled and stored at 0-4°C in the presence of 0.01% of sodium azide. Protein concentration was estimated using Bradford Reagent (from Biorad). Each sample was checked for purity by electrophoresis on 10% SDS - polyacrylamide gel stained with Coomassie blue.

Assay procedure

The IgG samples purified in the above manner were diluted with phosphate buffered saline (hereinafter PBS, pH 7.2, containing 8 g NaC1, 0.2 g KC1, 0.2 g potassium dihydrogen orthophosphate and 1.15 g dipotassium hydrogen orthophosphate/litre) to concentrations of 20 $\mu$g IgG and 40 $\mu$g IgG per 100 $\mu$l. A nitrocellulose membrane (Schleicher and Schuell, 0.1 $\mu$m pores) was prepared with a pencil grid (0.8 X 0.8 cm squares) and a suitable border for handling and then placed on twelve layers of absorbent tissue. The IgG samples were dot blotted onto the nitrocellulose. 5 $\mu$l of each IgG sample was applied using a 10 $\mu$l Hamilton syringe and Chaney adaptor. The application was arranged so that the needle of the syringe does not touch the nitrocellulose. The solution was carefully applied to reduce lateral movement of the solution along the membrane so that it was caused to pass through the nitrocellulose into the absorbent tissue. Each sample (including standards used for comparison) was applied in triplicate using 1 $\mu$g IgG/5 $\mu$l when abrin or ricin was used as the lectin probe and 2 $\mu$g IgG/5 $\mu$l when Bandeiraea lectin was used. The dot blots were then dried at room temperature for one hour after which they were boiled in PBS for five minutes to denature the IgG molecule and expose the sugar residues. The nitrocellulose membrane was then treated for one hour at room temperature (or at 0-4°C overnight) with 1% bovine serum albumin (BSA) in PBS containing 0.5% Tween (Trade Mark) wetting agent. (The bovine serum albumin reduces non-specific protein:protein interactions and the Tween wetting reagent reduces non-specific background binding to the nitrocellulose).

The lectin probes were prepared by reacting each lectin with N-hydroxysuccinimidobiotin. The lectin in a concentration of 1 mg/ml in distilled water was treated with the biotinylating reagent at a strength of 1 mg/ml in dimethylsulphoxide in a ratio of biotin:lectin of 1:10 and left overnight at room temperature. The biotinylated lectin obtained was stored at 0-4°C in the presence of 0.01% of sodium azide.

The nitrocellulose membrane carrying the dot blots was incubated for two and a half hours with the biotinylated lectin. [The ricin-based reagent, RCAII B chain, was used at a dilution of 1/250, the abrin-based reagent at 1/125, and the Bandeiraea lectin based reagent at 1/100, all diluted with PBS containing BSA and Tween.] The blots were then washed five times at ten minute intervals with PBS containing Tween and BSA. They were then incubated for two hours on a rotary shaker with Streptavidin labelled with Horse Radish Peroxidase at 1/1000 in PBS-Tween-BSA. The blots were washed five times at ten minute intervals with PBS containing Tween only, and then developed with 4-chloro-naphthol and hydrogen peroxide. To one volume of methanol was added 0.3 mg/ml of 4-chloro-1-naphthol followed by five volumes of Tris buffer (pH 7.6) and 0.01% of hydrogen peroxide (0.1% for Bandeiraea lectin treated blots). The mixture was prepared immediately before use. After the colour had formed, the membrane was washed with distilled water and the blots were dried thoroughly. Their densities were then read with a photometer.

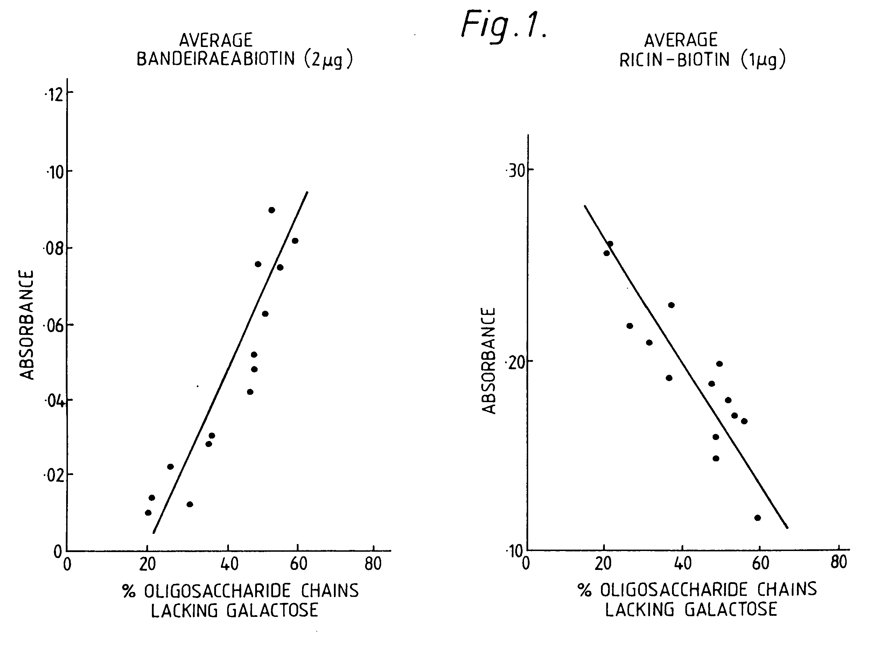

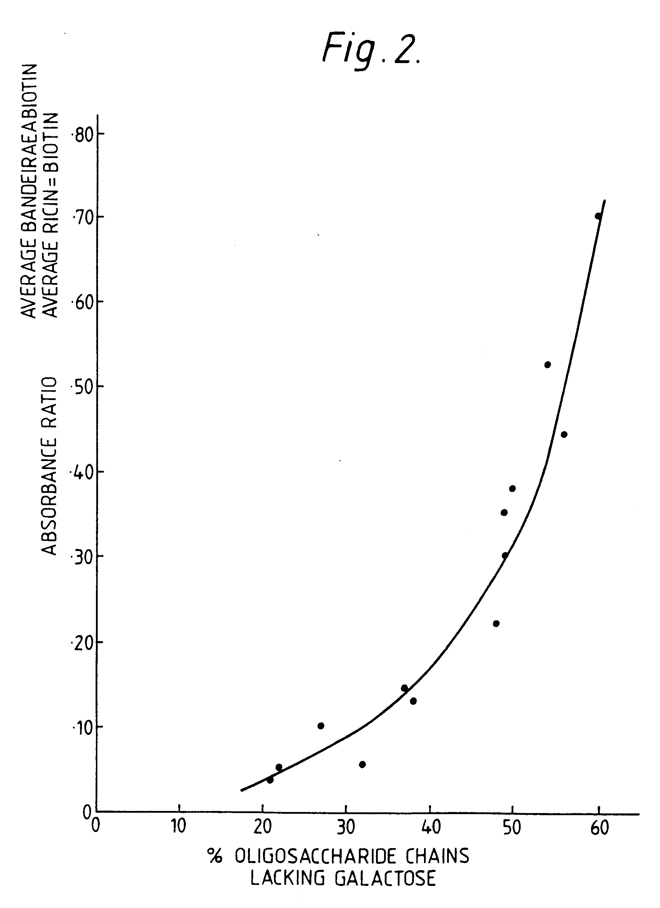

Figure 1 shows the relationship between absorbance measured with the photometer and percentage oligosaccharide chains lacking galactose. The latter were obtained by quantitative biochemical analysis using the method described by Parekh et al J.Exp. Med., 167, 1731-1736, 1988. The excellent correlation between the new method and the Parekh et al method is evident. Figure 2 shows a plot of the absorbance ratio of dots measured using Bandeiraea lectin to dots measured using ricin, i.e. a measure of the ratio of terminal N-acetylglucosamine residues to terminal galactose residues. It will be noted that the new method provides a very sensitive measure of the percentage of oligosaccharide chains lacking galactose in the range 20-60%. The dot blot assay can be modified and performed on polystyrene ELISA plates as illustrated in Example 2.

## Example 2

The purified samples of human IgG to be analysed were treated with 8M urea containing 0.5M mercaptoethanol (Sample/urea ratio + 2mg/2ml) and incubated overnight at 37°C. The treated samples were then dialysed overnight against 0.02M iodoacetamide, followed by dialysis against PBS for 2h. This first step of treating samples with urea is only necessary if the samples need to be denatured to expose the carbohydrates. A human IgG sample is believed to require denaturation.

Protein estimation was carried out on the denatured human IgG samples. Elisa plates were coated with the sample ($100\mu$l of sample per well) at a protein concentration of $10\mu$g/ml in carbonate/bicarbonate buffer, pH 9.6. The plates were left at 4°C overnight or 3h at room temperature after which they were washed three times with PBS followed by blocking with PBS-Tween (0.05%)-BSA (1%) for 1h at room temperature. After washing three times with PBS, the biotinylated reagents (100 $\mu$l/well) used in the dot blot assay of Example 1 were added and the plates incubated overnight at 4°C.

The diluent for the reagents was PBS-Tween-BSA 0.1%.

The plates were washed again 3 times with PBS-Tween and streptavidin-HRP conjugate ($100\mu$l/well) added. The plates were incubated for 2h at room temperature followed by washing three times with PBS-Tween.

Substrate was then added to the wells and the colour developed in the dark for 10-30 minutes. The reaction was stopped using sodium fluoride (96mg/50ml $H_2O$) and the plates read at 405nm.

The substrate used was 2'2' Azino-di(3,ethyl benzthiazoline sulfonic acid in 0.05M citrate phosphate buffer, pH4.1. 50mg substrate was present in 100ml citrate phosphate buffer + $35\mu$l $H_2O_2$ vol 20.

The results obtained for human IgG samples are given in Table 1.

## Table 1

### Analysis of human IgG glycosylation using an Elisa assay

| %Go | Binding to Galactose | Binding to GlcNac | Ratio of Gal /GlcNac binding/ binding |
|---|---|---|---|
| 21 | 0.677 | 0.392 | 0.78 |
| 32 | 0.509 | 0.643 | 1.18 |
| 38 | 0.430 | 0.750 | 2.25 |
| 61 | 0.107 | 0.472 | 6.27 |

The degree of galactose deficiency (Go) is shown to be related to the ratio in the final column and this method of measuring the terminal glycosylation pattern is believed to be particularly sensitive and versatile.

## Claims

1. A method for detecting and/or measuring the terminal glycosylation pattern of human IgG characterized in that it comprises treating samples of IgG which have been treated to expose terminal sugar residues with a corresponding plural number of labelled lectins at least one of which has a specific binding affinity for galactose and at least one other of which has a specific binding affinity for N-acetyl-glucosamine, each such lectin being used in excess, removing unbound labelled lectin from the said samples, and then observing and/or measuring the signal provided by each bound or unbound lectin.

2. A method according to claim 1 characterised in that the lectins binding terminal galactose residues are from Abrus precatorius or Ricinus communis, and the lectins binding terminal N-acetylglucosamine residues are from Bandeiraea simplicifolia, Datura stramonium or Lyconersicon esculentum.

3. A method according to claim 1 or 2 characterised in that the lectins are each enzyme labelled.

4. A method according to claim 1, 2 or 3 characterised in that the samples of human IgG to be examined are bound to solid substrate prior to treatment with the labelled reagents.

5. A method according to claim 4 characterized in that the IgG is denatured by boiling after binding to the solid substrate or by disulphide bond reduction and treatment with 8M urea before binding to the solid substrate.

6. A method according to claim 4 or 5 characterised in that the solid substrate is nitrocellulose.

7. A method according to claim 4 or 5 characterised in that the solid substrate is a polystyrene.

8. A kit suitable for carrying out the method of any one of claims 1 to 7, characterized by comprising a solid substrate onto which the IgG may be bound, and a number of vials each containing a labelled lectin having a specific binding affinity for a terminal galactose or a terminal N-acetylglucosamine residue.

## Patentansprüche

1. Verfahren zur Feststellung und/oder Meßung der endständigen Glycosylationsmuster von humanem IgG, dadurch gekennzeichnet, daß es umfaßt die Behandlung von IgG-Proben, die behandelt wurden, um endständige Zuckerreste freizulegen, mit einer entsprechenden mehrfachen Zahl gekennzeichneter Lektine, von denen wenigstens eines eine spezifische Bindungsaffinität für Galactose und wenigstens ein anderes eine spezifische Bindungsaffinität für N-Acetylglucosamin hat, wobei jedes dieser Lektine im Überschuß verwendet wird, das Entfernen von ungebundenem gekennzeichneten Lektin aus den Proben und das Beobachten und/oder Meßen des von jedem gebundenen oder ungebundenen Lektin stammenden Signals.

2. Verfahren nach Anspruch 1, ddurch gekennzeichnet, daß die Lektine bindenden endständigen Galactosereste von Abrus precatorius oder Ricinus communis und die Lektine bindenden endständigen N-Acetylglucosamin-Reste von Bandeiraea simplicifolia, Datura stramonium oder Lycopersicon esculentum stammen.

3. Verfahren nach Anspruch 1 oder 2, dadurch gekennzeichnet, daß die Lektine durch Enzyme gekennzeichnet sind.

4. Verfahren nach Anspruch 1, 2 oder 3, dadurch gekennzeichnet, daß die zu untersuchenden Proben des humangen IgG vor der Behandlung mit den gekennzeichneten Reagenzien an festes Substrat gebunden werden.

5. Verfahren nach Anspruch 4, dadurch gekennzeichnet, daß das IgG nach dem Binden an das feste Substrat durch Kochen oder vor dem Binden an das feste Substrat durch Reduktion der Disulfidbindung und Behandlung mit 8M Harnstoff denaturiert wird.

6. Verfahren nach Anspruch 4 oder 5, dadurch gekennzeichnet, daß das feste Substrat Nitrocellulose ist.

7. Verfahren nach Anspruch 4 oder 5, dadurch gekennzeichnet, daß das feste Substrat ein Polystyrol ist.

8. Ausrüstung zur Durchführung des Verfahrens eines der Ansprüche 1 bis 7, dadurch gekennzeichnet, daß sie ein festes Substrat, auf dem das IgG gebunden werden kann, und eine Zahl von gläsernen Fläschchen umfaßt, von denen jedes ein gekennzeichnetes Lektin mit einer spezifischen Bindungsaffinität für eine endständige Galactose oder einen endständigen N-Acetylglucosamin-Rest hat.

**Revendications**

1. Méthode pour la détection et/ou la mesure du motif terminal de glycosylation de l'IgG humaine, caractérisée par le fait qu'elle comprend le traitement d'échantillons d'IgG qui ont été traités afin d'exposer les résidus sucres terminaux à un nombre correspondant de lectines marquées dont l'une au moins a une affinité spécifique de liaison pour le galactose et une autre au moins a une affinité spécifique de liaison pour la N-acétylglucosamine, chacune de ces lectines étant utilisée en excès, l'élimination des lectines marquées non fixées desdits échantillons puis l'observation et/ou la mesure du signal produit par chaque lectine fixée ou non.

2. Méthode selon la revendication 1, caractérisée par le fait que les lectines fixant les résidus galactose terminaux proviennent de Abrus precatorius ou de Ricinus communis et les lectines fixant les résidus N-acétylglucosamine terminaux proviennent de Bandeiraea simplicifolia, de Datura stramonium ou de Lycopersicon esculentum.

3. Méthode selon la revendication 1 ou 2, caractérisée par le fait que les lectines sont marquées chacune par une enzyme.

4. Méthode selon la revendication 1, 2 ou 3, caractérisée par le fait que les échantillons d'IgG humaine à examiner sont fixés à un substrat solide antérieurement au traitement avec les réactifs marqués.

5. Méthode selon la revendication 4, caractérisée par le fait que l'IgG est dénaturée par ébullition après fixation au substrat solide ou par réduction des liaisons disulfure et traitement avec de l'urée 8M avant la fixation au substrat solide.

6. Méthode selon la revendication 4 ou 5, caractérisée par le fait que le substrat solide est la nitrocellulose.

7. Méthode selon la revendication 4 ou 5, caractérisée par le fait que le substrat solide est un polystyrène.

8. Trousse adaptée à la mise en oeuvre de la méthode selon l'une quelconque des revendications 1 à 7, caractérisée par le fait qu'elle comprend un substrat solide sur lequel l'IgG peut être fixée, et un nombre de fioles contenant chacune une lectine marquée ayant une affinité spécifique de liaison pour un résidu galactose terminal ou pour un résidu N-acétylglucosamine terminal.

Fig.1.

AVERAGE
BANDEIRAEABIOTIN (2µg)

AVERAGE
RICIN-BIOTIN (1µg)

% OLIGOSACCHARIDE CHAINS
LACKING GALACTOSE

% OLIGOSACCHARIDE CHAINS
LACKING GALACTOSE

EP 0 386 092 B1

# Fig. 2.